# NOUVEAU FASCICULE DE BREVET EUROPEEN

(11) **EP 1 395 649 B2**
(45) Date de publication et mention de la décision concernant l'opposition: **09.03.2016**
(45) Mention de la délivrance du brevet: 22.10.2008
(21) Numéro de dépôt: 02745494.1
(22) Date de dépôt: 07.06.2002
(51) Int. Cl.: C12N 1/18, C12G 1/02, C12N 1/04

(54) **PROCEDE DE REHYDRATATION DE LEVURES SECHES ACTIVES, ET MILIEU DE REHYDRATATION**
VERFAHREN ZUR REHYDRATATION VON AKTIVEN TROCKENEN HEFEN UND REHYDRATATIONSMEDIUM
METHOD FOR ACTIVE DRY YEAST REHYDRATION, AND REHYDRATION MEDIUM

(30) Priorité: 08.06.2001 FR 0107535
(43) Date de publication de la demande: 10.03.2004
(73) Titulaire: Danstar Ferment AG, 6300 Zug (CH)
(72) Inventeur: DULAU, Laurent, F-31000 Toulouse (FR); ORTIZ-JULIEN, Anne, F-31200 Toulouse (FR); TRIOLI, Gianni, I-29028 Ponte dell'Olio (IT)
(74) Mandataire: Cabinet Armengaud Aîné
(86) Numéro de dépôt international: PCT/FR2002/001949
(87) Numéro de publication internationale: WO 2002/101024

(56) Documents cités:
- EP-A- 0 008 554
- EP-A- 0 616 030
- WO-A-91/18513
- FR-A- 2 607 147
- GB-A- 1 457 123
- MORETTI S: "Effect of maintenance technique and reproduction of selected yeast cultures on metabolic activity." ENOTECNICO 1992 ISTITUTO SPERIMENTALE PER L'ENOLOGIA, VELLETRI, ITALY, vol. 28, no. 5, pages 79-83, XP001122801
- F. RADLER ET AL: "Mikrobiologische Prüfung von Trockenhefepräparaten für Weinbereitung" DEUTSCHE LEBENSMITTEL-RUNDSCHAU, vol. 3, 1985, pages 73-77, XP008001482
- J.K. KRAUS ET AL: "Levures sèches actives de vinification. 1re partie: Fabrication et caractéristiques" CONNAISSANCE DE LA VIGNE ET DU VIN, vol. 17, no. 2, 1983, pages 93-103, XP001061335 cité dans la demande
- M.J. BEKERS ET AL: "Conservation of yeasts by dehydration" ADVANCES IN BIOCHEMICAL ENGINEERING/ BIOTECHNOLOGY, vol. 35, - 1987 pages 127-171, XP008001378
- F. ZÜRN ET AL: "Untersuchungen zur Reaktivierung von Trockenhefen" WEINWIRTSCHAFT, vol. 34, 2 octobre 1981 (1981-10-02), pages 1010-1012, XP008001484
- J.K. KRAUS ET AL: "Effect of rehydration on dry wine yeast activity" AM, J. ENOL. VITIC., vol. 32, no. 2, 1981, pages 132-134, XP001061225
- P. MONK: "Optimum usage of active dried wine yeast" PROCEEDINGS OF THE ASVO SEMINAR; ADVANCES IN JUICE CLARIFICATION AND YEAST INOCULATION. MELBOURNE , AUSTRALIA, 1997, pages 22-23, XP001058469 cité dans la demande
- R. DEGRE: "Selection and commercial cultivation of wine yeast and bacteria" WINE MICROBIOL. BIOTECHNOL. EDITOR(S): FLEET, GRAHAM H. PUBLISHER: HARWOOD, CHUR, SWITZ., 1993, pages 421-447, XP001066551
- SIGMA-ALDRICH: " FLUKA CHEMIKA-BIOCHEMIKA CATALOG " 1997 , SIGMA-ALDRICH COMPANY , BUCHS, SWITZERLAND XP002215953 page 1593 -page 1594
- B.C. RANKINE: "modern developments in selection and use of pure yeast cultures for wine making" AUSTRALIAN WINE, BREWING AND SPIRIT REVIEW, vol. 96, no. 9, 27 juillet 1977 (1977-07-27), pages 32-34, XP001061333
- H. ALEXANDRE ET AL: "Biochemical aspects of stuck and sluggish fermentation in grape must" JOURNAL OF INDUSTRIAL & BIOTECHNOLOGY, vol. 20, 1998, pages 20-27, XP001053543 cité dans la demande

## Description

La présente invention concerne le domaine technique de la production de boissons alcoolisées, en particulier celui de la production de vins. Dans ce domaine, les problèmes associés aux fermentations représentent un manque en gagner important en termes économiques. En effet ces problèmes fermentaires se traduisent, au niveau des vins concernés, par des pertes en rendement, en qualité, voire parfois par des pertes en volume. Il est donc important pour les entreprises de cette filière de diminuer la fréquence d'apparition des problèmes fermentaires.

De manière maintenant répandue, des cultures pures de levures sont utilisées pour permettre un départ rapide et un déroulement régulier de la fermentation des moûts de raisin. Les cultures pures de levures de vinification sont des souches sélectionnées de la famille des *Saccharomyces* appartenant généralement aux espèces *cerevisiae, uvarum* ou *bayanus*.

Cependant, un des moments critiques de la fermentation reste la fin de cette fermentation. Il est essentiel de pouvoir terminer cette réaction avec une vitesse de consommation des sucres suffisante afin d'éviter des situations d'arrêt ou de fermentations languissantes qui représentent des situations à risque, d'autres micro-organismes, notamment des bactéries lactiques, pouvant alors coloniser rapidement le milieu fermentaire en stoppant définitivement la fermentation alcoolique. Certains de ces microorganismes utiliseront les sucres résiduels pour produire des métabolites indésirables comme par exemple l'acide acétique, entraînant des pertes de qualité au niveau du vin concerné.

On entend par fermentations problématiques les fermentations correspondant à deux types de situations : les fermentations lentes et les fermentations languissantes. La vitesse de fermentation d'un milieu est définie comme la quantité de dioxyde de carbone dégagée par unité de temps. Elle est représentée par la courbe dérivée de la quantité de dioxyde de carbone dégagée en fonction temps V = dCO₂/dt.

Dans les fermentations lentes, les vitesses maximales observées au cours de la fermentation sont peu élevées. Ces fermentations lentes sont généralement dues à des populations levuriennes présentant une activité métabolique faible, généralement associées à une carence en azote assimilable des moûts.

Les fermentations languissantes ou les arrêts de fermentation sont caractérisées par une vitesse maximale de fermentation relativement élevée, mais qui diminue progressivement, la viabilité des levures devenant très faible. Ces fermentations ralentissent alors fortement ou s'arrêtent totalement lorsque la population levurienne est insuffisante pour assurer l'achèvement de la consommation des sucres. Dans la majorité des cas, ce type de phénomène est observé lors de carences en oxygène ou de fortes carences en azote.

Il est bien connu que ces fermentations problématiques sont le plus souvent associées à des déséquilibres ou des carences des milieux nutritifs (Alexandre et al. J. of Ind. Microbiol. and Biotechnology, vol. 20, 1998: pages 20-27). En effet, différents nutriments sont nécessaires pour permettre aux levures, d'une part de bien se développer afin de coloniser le milieu fermentaire et d'autre part d'assurer efficacement le métabolisme des sucres en alcool et ce, jusqu'à l'épuisement total de ces sucres. Ces nutriments appartiennent notamment aux catégories suivantes : les sources d'azote, les sources d'oxygène, les vitamines, les sels minéraux.

L'azote assimilable, est constitué par l'azote ammoniacal et l'azote α-aminé. C'est le nutriment qui influe le plus sur la vitesse de la fermentation alcoolique (Agenbach. Proc. South African Soc. Enol. Vitic., Cape town, South Africa. Stellenbosh SA, 1977 : pages 66-87). L'azote est un élément essentiel puisqu'il permet la synthèse des protéines, et donc la croissance des levures. Sa carence dans le moût, d'une part limite la croissance levurienne et donc la vitesse de fermentation, et d'autre part induit une diminution importante de l'activité de transport des sucres, augmentant ainsi les risques d'arrêt de fermentation ou de fermentation languissante. Une carence en azote peut aussi entraîner une déviation du métabolisme de la levure, conduisant notamment à la production de sulfure d'hydrogène (H₂S), néfaste aux caractéristiques aromatiques du vin.

En général, les moûts en contiennent entre 80 et 400 mg/l alors que le seuil de carence se situe vers 150-180 mg/l. Une carence azotée du moût est donc un risque majeur pour le bon déroulement de la vinification.

L'oxygène joue un rôle primordial sur la fermentation alcoolique. Il assure le bon développement de la population levurienne et le maintien d'une bonne viabilité des levures. Il permet la synthèse de composés lipidiques tels que les stérols ou les acides gras insaturés constituant la membrane cellulaire des levures et entraînant une meilleure résistance à l'éthanol. La fluidité membranaire est en effet fonction du ratio acides gras insaturés / acides gras saturés. La présence d'acides gras insaturés est donc primordiale pour cette résistance à l'alcool.

Les besoins minimaux en O₂ pour le bon déroulement d'une fermentation peuvent être estimés à environ 5 -10 mg /l (Sablayrolles et Barre. Sci. Alim., vol. 6, 1986 : pages 373-383). Cependant, l'oxygène est très rapidement consommé en début de fermentation d'une part par les enzymes (cas de pourriture : laccase), et d'autre part par la flore levurienne oxydative. Les viticulteurs sont donc amenés à ajouter de l'oxygène en cours de fermentation, avant épuisement du milieu. Le moment de l'ajout de cet élément (mi-fermentation, après multiplication des cellules et dilution des lipides de la flore fermentaire) est donc primordial.

Un autre moyen connu pour éviter les problèmes de fermentation liés à une carence en oxygène est l'ajout au moût d'acides gras insaturés, de stérols ou de composés riches en ces molécules tels que les bourbes et certains dérivés de levures.

Certaines vitamines jouent un rôle essentiel sur le déroulement de la fermentation alcoolique. La biotine par exemple favorise la production d'esters et permet une meilleure viabilité cellulaire en fin de fermentation. En cas de carence, la croissance cellulaire est significativement affectée. Le pantothénate, impliqué dans le métabolisme des lipides, a un effet positif sur les qualités organoleptiques : il diminue les risques de production d'H₂S ainsi que d'acidité volatile. La thiamine enfin, dont le rôle a fait l'objet de nombreuses études, est une autre des vitamines qui peut être limitante et dont la carence peut provoquer des arrêts de fermentation. Ce déficit est souvent du à certains traitements préfermentaires réalisés dans des conditions mal maîtrisées (sulfitage, chauffage). En effet, la thiamine possède la propriété de se combiner rapidement au SO₂ et n'est alors plus biodisponible. Elle peut en plus disparaître rapidement du milieu. Il a effectivement été démontré (Bataillon et al., J. Ferm. Bioeng., vol. 82, 1996 : pages 145-150) qu'en présence de 106 cellules de *Saccharomyces cerevisiae* / ml, la quasi-totalité de la thiamine a disparu en 2 à 3 heures, les levures pouvant en accumuler des concentrations importantes. De plus, la flore indigène non levurienne consomme cet élément parfois plus rapidement que *Saccharomyces cerevisiae*.

D'autres nutriments tel que le magnésium, le zinc, le manganèse ou le potassium sont également très importants pour l'activité de la levure. Le magnésium en particulier joue un rôle essentiel dans le contrôle de la croissance cellulaire et le métabolisme des levures. Il permet une meilleure résistance à la température et à la pression osmotique. Il intervient au niveau du maintien de l'intégrité cellulaire (stabilisation des acides nucléiques, des polysaccharides, lipides et protéines) (Walker, Critic. Rev. Biotechnol., vol. 14, 1994: pages 311-354). Des ajouts de magnésium augmentent la production d'éthanol. Le zinc est aussi très important car c'est un co-facteur des enzymes de la glycolyse ; il permet une meilleure tolérance à l'alcool et joue un rôle important dans la production d'esters.

Bien que les carences en ces éléments minéraux dans le milieu de fermentation soient peu fréquentes, ils ne sont que rarement présents sous forme biodisponible. En effet ces cations pouvant être liés à différents types de molécules dans le moût, telles que les protéines et les polyphénols, la levure ne peut les utiliser.

La composition des moûts en nutriments utiles pour la levure est donc considérée comme une caractéristique cruciale pour la bonne conduite de la vinification. L'éventualité d'une carence en l'un ou en l'autre fait courir un risque important quant au produit qui sera obtenu en fin de compte. C'est pourquoi, pour pallier ces risques de carence, certaines solutions ont été proposées et sont actuellement pratiquées.

Aujourd'hui plusieurs pratiques sont mises en oeuvre afin de complémenter les moûts carencés tout au long de la fermentation et de diminuer la fréquence d'apparition de fermentations problématiques:
- Pour les carences en azote : l'ajout dans le moût à mi-fermentation de phosphate di-ammonique ou de sulfate d'ammonium, est préconisé (Sablayrolles et al., J. Ferm. Bioeng., vol. 82, 1996 : pages 377-381).
- Pour les carences oxygène et en facteurs de croissance, l'ajout dans le moût de bourbes fines permet de maîtriser la turbidité et d'apporter des nutriments tels que les stérols et les acides gras insaturés (Delfini et al. Am. J. Enol. Vitic., vol. 44, 1993 : pages 86-92). Toutefois, cet ajout n'est pas toujours facile à réaliser dans la pratique, dans les caves. C'est pourquoi, l'ajout de produits complexes contenant des levures inactivées a été proposé afin de compenser les carences du moût. Ces produits contiennent généralement, en plus des levures inactivées ou des écorces de levures, du phosphate d'ammonium et de la thiamine. Outre leurs apports nutritifs, ils influent sur la qualité globale des vins. L'efficacité de l'addition de tels produits a été démontrée dans de nombreuses situations oenologiques (Trioli, Vitic. Enol. Sci., vol. 51 (3), 1996 : pages 204-209).

Récemment, un moyen d'intervention particulièrement efficace a été développé afin de diminuer les risques d'arrêts de fermentation (Sablayrolles et al. J. Ferm. Bioeng. vol. 82, 1996 : pages 377-381). Il consiste à réaliser simultanément un apport dans le moût d'azote et d'oxygène. On constate alors que les effets s'additionnent. Il est cependant primordial que ces ajouts combinés soient maîtrisés, le moment des apports et la quantité des nutriments apportés étant essentiels.

Enfin l'addition de thiamine est une pratique courante. De façon commune elle est ajoutée en début de fermentation ce qui la rend très peu disponible pour la levure chargée d'assurer la fermentation alcoolique.

A noter par ailleurs, que des études récentes sur la caractérisation des besoins en azote assimilable et oxygène de différentes levures fermentaires permettent au professionnel de choisir sa levure en fonction de la qualité nutritive du moût qu'il a à fermenter (Julien et al., Am. J. Enol. Vitic., vol. 51 (3), 2000 : pages 215-222). Cependant cette connaissance ne permet pas de s'affranchir de la correction des moûts en certains facteurs nutritifs.

Ainsi toutes les solutions techniques décrites et utilisées jusqu'à présent reposent sur le même principe : réaliser un ou plusieurs apports dans le moût des nutriments indispensables à l'activité fermentative de la levure en début ou au cours de la fermentation.

Ces méthodes ne permettent pas de fournir directement à la levure les nutriments utiles en quantité et/ou concentration suffisante pour maintenir une vitesse de fermentation élevée jusqu'au terme de la fermentation, ni de s'affranchir du problème de disponibilité de certains éléments essentiels au bon développement de la levure fermentaire.

En conséquence, malgré l'application de ces techniques il existe encore un grand nombre de situations de fermentation problématique.

Ces vingt dernières années, l'emploi de levains sélectionnés sous forme sèche (Levures Sèches Actives, LSA) s'est généralisé. Avant leur utilisation, il est nécessaire d'opérer une étape de réhydratation. Cette étape, d'une relative simplicité de mise en oeuvre, est cependant capitale pour la qualité du levain une fois réhydraté (Monk, Proceedings of the ASVO Seminar : Advances in Juice Clarification and Yeast Inoculation. Melbourne, Aus. 22-23, 1997).

De nombreux auteurs ont fourni les bases théoriques et ont décrit les transformations structurelles et biochimiques qui s'opèrent dans les cellules de levure lors des opérations de déshydratation et réhydratation (M.J. Bakers et al., Advances in Biochemical Engineering / Biotechnology, vol. 35, 1987, pp. 127-171). Les conditions optimales à mettre en oeuvre lors de la réhydratation pour éviter autant que possible la destruction des cellules ont également fait l'objet de diverses études. L'une des plus complètes a été réalisée par Radler *et al.* sur la réhydratation des levures de vinification (Von F. Radler et al., Deutsche Lebensmittel-Rundschau, vol 3, 1985, pp. 73-77). Il a été ainsi mis en évidence que la composition du milieu de réhydratation avait une influence sur l'activité métabolique de la levure mesurée durant les deux premières heures après la réhydratation. En particulier les mélanges de jus de raisin et d'eau et les solutions contenant une quantité définie de glucose, de fructose, d'acide malique, d'acides aminés, d'acide 2-cétoglutarique, de sulfate d'ammonium, de vitamines ou de KCl, de CaCl₂ et de NaCl ont eu un effet plus ou moins marqué sur l'activité métabolique des cellules de levure sèche, la plus forte augmentation de l'activité ayant été obtenue avec une solution de KCl à 1 %.

Cette étude, comme la plupart de celles réalisées dans ce domaine, avait pour objectif d'obtenir un taux de cellules revivifiées le plus élevé possible grâce à des conditions de réhydratation définies, le critère décisif étant la capacité de la levure à reprendre une activité fermentative élevée dans les plus brefs délais. La composition des milieux de réhydratation a été conçue en fonction de ce critère, testée sur des essais de courte durée, la nature des composants desdites milieux et les concentrations mises en oeuvre visant essentiellement à préserver l'intégrité de la membrane cellulaire et à stabiliser la pression osmotique, par exemple par l'ajout de sels.

Cependant, il n'a pas été mis en évidence que des conditions de réhydratation particulières des levures pouvaient avoir une influence à plus long terme sur l'activité fermentative des cellules de levure. En particulier, il n'a jusqu'à présent pas été suggéré qu'une solution permettant d'éviter les fermentations problématiques soit à rechercher dans les conditions de réhydratation des levures, c'est-à-dire avant leur introduction dans le moût à fermenter.

De manière surprenante et inattendue, il a été trouvé que la réhydratation des levures sèches actives (LSA) dans un milieu additionné d'un complexe nutritif comprenant des dérivés de levure et éventuellement d'autres nutriments, permettait d'augmenter les capacités fermentaires des levures issues de cette étape de réhydratation, notamment en fin de fermentation, alors que l'addition de ce type de complexe dans le moût ne permettait pas d'observer de tels effets.

Ainsi le procédé permet de prévenir les fermentations problématiques, notamment grâce à l'utilisation de milieux de réhydratation des levures sèches actives particuliers, ainsi qu'à l'application de ce procédé à la production de vins et autres boissons alcoolisées par fermentation.

Dans ce procédé de réhydratation de levures sèches actives pour la fermentation alcoolique lesdites levures sèches actives sont placées dans un milieu aqueux contenant au moins des levures inactives ou un dérivé de levure à raison de 100 à 200 g/l, de préférence 150g/e de milieu. Dans ce procédé, le milieu de réhydratation des LSA contient éventuellement un ou plusieurs nutriments complémentaires susceptibles de se trouver en concentration ou en disponibilité insuffisante dans le moût. Ces éléments nutritifs complémentaires sont choisis parmi les sources d'azote organique ou inorganique, les vitamines, les sels minéraux, les acides gras, les stérols, ou des sources naturelles riches en ces éléments.

Les levures sèches actives concernées sont toutes les levures de vinification employées pour ensemencer les moûts de raisins afin d'assurer un bon démarrage et un déroulement régulier de la fermentation. Il s'agit en particulier des levures de la famille des *Saccharomyces,* de préférence *Saccharomyces cerevisiae*.

Les levures inactives et les dérivés de levures sont bien connus de l'homme du métier et sont disponibles dans le commerce. Il est entendu que peuvent être utilisées dans le présent procédé des levures inactives enrichies en sels minéraux, par exemple telles que celles décrites dans la demande de brevet GB 98 1110.02, ou des levures inactives enrichies en tout autre nutriment. Les dérivés de levure sont tous les produits susceptibles d'être obtenus à partir de cellules de levure entières ou fractionnées, par action physique ou chimique. Ils comprennent en particulier les extraits de levure obtenus par autolyse et les écorces de levure. Ils se présentent le plus souvent sous forme de poudre plus ou moins fine après broyage et selon l'invention, ils sont employés en suspension dans le milieu de réhydratation.

Le milieu de réhydratation utilisé est, à la base, un milieu aqueux qui peut être sucré ou non. Dans le cas d'un milieu sucré, on utilise de préférence, comme il est couramment pratiqué, de l'eau glucosée ou du jus de raisin, prélevé en général sur le moût qu'on envisage de fermenter.

Les levures inactives ou dérivés de levure ainsi que les nutriments complémentaires sont introduits dans le milieu de base, puis les LSA sont ajoutées de manière que la réhydratation se déroule dès le départ dans le milieu tel que défini. Ainsi, le procédé de rehydratation de levures sèches actives selon l'invention comprend essentiellement les étapes consistant à:
a) préparer un milieu de réhydratation aqueux, sucré ou non sucré, comprenant
   i) des levures inactives ou un dérivé de levure à raison de 100 à 200 gl, de préférence 150 gl de milieu, et éventuellement
   ii) un ou plusieurs nutriments choisis parmi des sources d'azote organique comprenant des sels d'ammonium, des nitrates, l'urée, des acides aminés, des peptides, des protéines ou une source biologique riche en azote : des acides gras, des stérols, une combinaison de ceux-ci ou une source naturelle riche en ces éléments telle que notamment les bourbes : des vitamines comprenant la thiamine, la biotine, l'acide pantothénique, la niacine, la riboflavine, la pyridoxine, ou une source naturelle riche en vitamines : des sels minéraux comprenant des phosphates, des sels de zinc, de magnésium, de calcium, de potassium, de sodium, de fer, de cuivre, de manganèse ou une combinaison de ces sels minéraux, chaque nutriment choisi étant apporté à une concentration 200 à 1 000 fois supérieure, de préférence 500 fois supérieure, à celle qu'il aura dans le moût à fermenter, dans un milieu aqueux.
b) introduire les levures sèches actives dans le milieu de réhydratation ainsi préparé à raison de 50 à 150 g l, de préférence 100 g l de milieu.
(c) incuber le milieu de rehydratation concernant les levures sèches actives à une température comprise entre 30°C et 45°C, de préférence 37°C, durant 20 à 40 minutes, de préférence 30 minutes.

En d'autres termes, les LSA sont réhydratées dans un milieu bien plus concentré en nutriments que ne le sera le moût dans lequel elles sont destinées à développer leur activité fermentaire. Lors de l'ensemencement, l'inoculum contenant les cellules de LSA revivifiées et du milieu de réhydratation, apporte l'ensemble de ses composants au moût. Le facteur de dilution sera fonction des volumes respectifs milieu de réhydratation/milieu de fermentation. De manière inattendu, ce rapport est avantageusement fixé de manière à ce que les concentrations en nutriments complémentaires dans le moût soient du même ordre de grandeur que les concentrations usuellement introduites dans les moûts en début ou au cours de la fermentation. Par exemple, si C1 est la concentration désirée du nutriment N1 dans le moût, et que le volume d'ensemencement est de 20 ml pour un litre de moût, soit un facteur 500, alors le milieu de réhydratation devra contenir N1 à une concentration de l'ordre de 500 x C1.

Selon un mode de réalisation préféré de l'invention, le procédé comprend essentiellement les étapes consistant à :
a) Préparer un milieu de réhydratation comprenant.
   i) des levures inactives ou un dérivé de levure à raison de 100 à 200 g l, de préférence 150 g l de milieu, et
   ii) un ou plusieurs des composants suivants : 20 à 50 mg l de pantothénate de calcium. 0.15 à 0.30 mg l de biotine. 20 à 60 mg l de sel de zinc. 200 à 500 mg l de sel de magnésium. 2 à 5 mg l de sel de manganèse, dans un milieu aqueux.
b) introduire les levures sèches actives dans le milieu de réhydratation ainsi préparé à raison de 50 à 150 g l, de préférence 100 g l de milieu.
c) incuber le milieu de réhydratation contenant les levures sèches actives à une température comprise entre 30°C et 45°C, de préférence 37°C, durant 20 à 40 minutes, de préférence 30 minutes.

Un autre objet de la présente invention est un milieu de réhydratation de levures sèches actives, susceptible d'être employé dans le procédé décrit précédemment. Un tel milieu comprend au moins, dans un milieu de base aqueux des levures inactives ou des extraits de levure à raison de 100 à 200 g/l, de préférence 150 g/l de milieu.

De manière avantageuse, ledit milieu de réhydratation comprend au moins un milieu aqueux sucré ou non
i) des levures inactives ou un dérivé de levure, à raison de 100 à 200 g l, de préférence 150 g l de milieu.
ii) un ou plusieurs nutriments choisis parmi des sources d'azote organique comprenant des sels d'ammonium, l'urée, des acides aminés, des peptides, des protéines ou une source biologique riche en azote : des acides gras, des stérols, une combinaison de ceux-ci ou une source naturelle riche en ces éléments telle que notamment les bourbes : des vitamines comprenant la thiamine, la biotine, l'acide pantothénique, la niacine, la riboflavine, la pyridoxine, ou une source naturelle riche en vitamines : des sels minéraux comprenant des phosphates, nitrates, des sels de zinc, de magnésium, de calcium, de potassium, de sodium, de fer, de cuivre, de manganèse, ou une combinaison de ces sels minéraux, chaque nutriment choisi étant apporté à une concentration 200 à 1 000 fois supérieure, de préférence 500 fois supérieure, à celle qu'il aura dans le moût à fermenter.

Dans un mode de réalisation "préféré", le milieu de réhydratation de levures sèches actives comprend au moins, dans un milieu aqueux, de préférence de l'eau glucosée à raison de 50 g l ou du jus de raisin.
i) des levures inactives ou des extraits de levure, à raison de 100 à 200 g l, de préférence 150 g l,
ii) un ou plusieurs des composants suivants : 27 mg l de pantothénate de calcium, 0.2 mg l de biotine, 50 mg l de sel de zinc, 433 mg l de sel de magnésium, 4 mg l de sel de manganèse.

Est également un objet de la présente invention une composition sèches destinée à la préparation d'un milieu de réhydratation de levures sèches actives, comprenant au moins
i) des levures inactives ou un dérivé de levure, sous forme déshydratée à raison de 100 à 200 g/l, de préférence 150 g/l de milieu.
ii) un ou plusieurs nutriments complémentaires choisis parmi les sources d'azote organique ou inorganique, les vitamines, les sels minéraux, les acides gras, les stérols, ou des sources naturelles riches en ces éléments.

Ladite composition sèche peut en particulier contenir notamment
i) au moins 98 % en poids de levures inactives ou de dérivé de levure, sous forme déshydratée,
ii) un ou plusieurs nutriments choisis parmi les sels d'ammonium, les nitrates, l'urée, les acides amines, les peptides, les protéines ou une source biologique riche en azote; un acide gras, un stérol, une combinaison de ceux-ci ou une source naturelle riche en ces éléments telle que notamment les bourbes; la thiamine, la biotine, l'acide pantothénique, la niacine, la riboflavine, la pyridoxine, ou une source naturelle riche en vitamines; les phosphates, les sels de zinc, de magnésium, de calcium, de potassium, de sodium, de fer, de cuivre, de manganèse ou une combinaison de ces sels minéraux.

De manière avantageuse, elle pourra être constituée de
i) environ 99, 3 % en poids de levures inactives ou de dérivé de levure, sous forme déshydratée
ii) un ou plusieurs nutriments choisis parmi l'acide pantothénique, la biotine, un sel de zinc, un sel de magnésium, un sel de manganèse.

L'inoculum, préparé par le procédé de réhydratation des LSA selon l'invention ou à l'aide du milieu de réhydratation revendiqué, pourra être utilisé pour ensemencer un moût destiné à la production d'une boisson fermentée alcoolisée. Il sera introduit dans le moût en une quantité préalablement définie en fonction des concentrations en LSA, levure inactive ou dérivés de levure, et nutriments complémentaires introduits dans le milieu de réhydratation, selon les critères de dilutions définis précédemment, à savoir de 200 à 1 000 fois, de préférence 500 fois.

La présente invention trouvera une application naturelle dans la production d'une boisson fermentée alcoolisée à partir d'un jus de raisin, telle qu'un vin.

Les exemples suivants illustrent de façon non limitative, des modes de réalisation de la présente invention.

### EXEMPLES

### Exemple 1 : Amélioration du profil de fermentation d'un moût synthétique par incorporation d'un extrait de levure dans le milieu de réhydratation de la levure fermentaire

Trois fermentations ont été conduites en parallèle et en duplicata sur un moût synthétique MS 70-fa (tel que décrit dans Bely *et al.* (1990) correspondant à un milieu carencé en azote assimilable (100 mg/l) et contenant 200 g/l de sucres fermentescibles. La levure sèche active utilisée est la levure commerciale Lalvin EC1118™.

Milieux de réhydratation
Mo : Milieu de réhydratation témoin : eau additionnée de 50 g/l de glucose.
Me : Milieu de réhydratation contenant un extrait de levures (Bacto-yeast extract ref. 0127-01-7, DIFCO, USA), utilisé à la dose permettant une concentration en extrait finale dans le moût de fermentation de 30g/hl, soit 150 g/l de milieu de réhydratation.

### Réhydratation

1- Un gramme de la levure sèche active Lalvin EC1118™ est réhydraté dans 10 ml de milieu Mo à 37°C pendant 30 minutes.
2- Un gramme de la levure sèche active Lalvin EC1118™ est réhydraté dans 10 ml de milieu Me à 37°C pendant 30 minutes.

### Fermentations

Fo : Fermentation témoin conduite avec la levure réhydratée dans le milieu Mo. Aucun extrait de levure n'est présent.
F1 : Fermentation conduite avec la levure réhydratée dans le milieu Mo. Un extrait de levure est rajouté au début de la fermentation alcoolique directement dans le moût synthétique à raison de 30g/hl.
F2 : Fermentation conduite avec la levure réhydratée dans le milieu Me contenant l'extrait de levure qui se retrouve à hauteur de 30 g/hl dans le moût synthétique.

Trois fermenteurs contenant 1,1 litres de moût synthétique MS70-fa, sont inoculés avec 2,2 ml de solution de réhydratation, (ce qui correspond à une dose d'utilisation de 20 g/hl de levure sèche active). La température de fermentation est de 24°C. La production de CO₂ est relevée en fonction du temps.

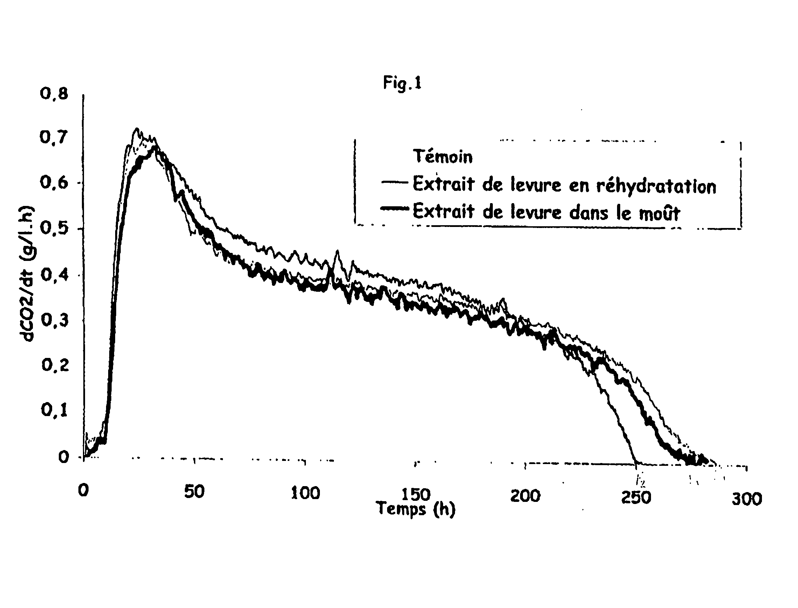

Les résultats obtenus (figure 1) montrent que la fermentation conduite par la levure réhydratée en présence d'extrait de levure dans le milieu de réhydratation (modalité F2) se termine avant les deux autres fermentations (modalités Fo et F1). La cinétique de fin de fermentation caractérisée par la pente de la courbe est plus rapide dans la modalité F2 expliquant le gain de temps observé d'environ 50 heures par rapports aux autres modalités. L'ajout d'extrait de levure, au début de la fermentation alcoolique, ne permet pas dans ces conditions, d'améliorer le profil fermentaire par rapport à la modalité témoin Fo.

### Exemple 2 : Amélioration du profil de fermentation d'un moût réel de Chardonnay par incorporation d'un extrait de levure dans le milieu de réhydratation de la levure fermentaire

Deux fermentations sont conduites en parallèle et en duplicata sur un moût réel de Chardonnay provenant du sud de la France (région Languedoc) contenant 365 mg/l d'azote assimilable (situation de non carence) et contenant 220 g/l de sucres fermentescibles. La levure sèche active utilisée est la levure commerciale Lalvin EC1118™, inoculée à la dose de 20 g/hl.

### Réhydratation

Un gramme de la levure sèche Lalvin EC1118™ est réhydraté dans 10 ml d'eau glucosée (50 g/l) à 37°C pendant 30 minutes. Dans la réhydratation témoin, aucun ajout n'est pratiqué. Dans la modalité 2, 1,5 g d'extrait de levure (Bacto-yeast extract ref. 0127-01-7, DIFCO, USA) est ajouté aux 10 ml du milieu de réhydratation.

### Fermentation

Les deux fermentations correspondent aux modalités suivantes :
F1 : Fermentation conduite avec la levure EC1118™ réhydratée dans un milieu de réhydratation en absence d'extrait de levure. L'extrait de levure est ajouté au début de la fermentation alcoolique directement dans le moût de chardonnay à la dose de 30 g/hl.
F2 : Fermentation conduite avec la levure EC1118™ réhydratée en présence d'un extrait de levure (utilisé à la dose permettant une concentration de 30g/hl dans le moût) dans le milieu de réhydratation.

Les fermenteurs de 1,1 litres sont inoculés avec 2,2 ml de solution de réhydratation contenant les LSA, ce qui correspond à une dose d'inoculation dans le moût de 20g/hl de levures sèches actives. La température de fermentation est de 24°C. La production de CO₂ est relevée en fonction du temps.

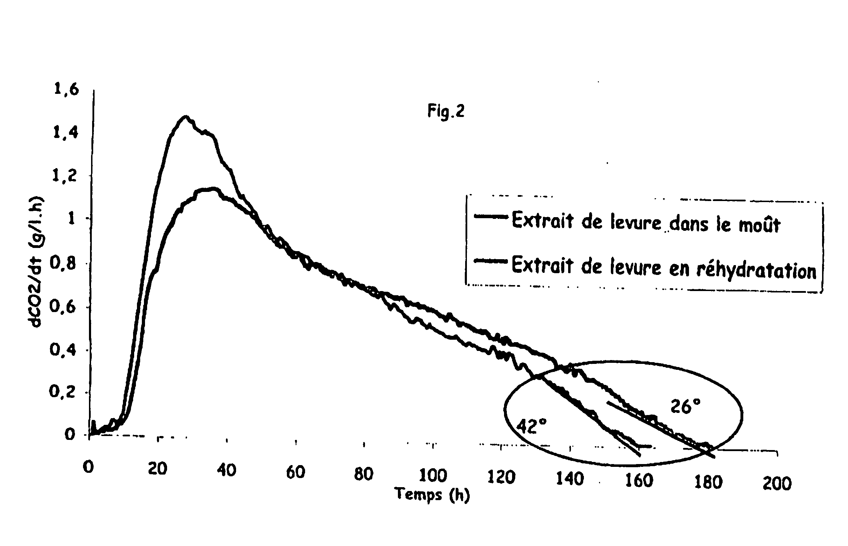

Les résultats obtenus (figure 2) montrent que la fermentation conduite par la levure réhydratée en présence d'extrait de levure dans le milieu de réhydratation (modalité F2) se termine avant la fermentation témoin F1. La cinétique de fin de fermentation caractérisée par la pente de la courbe est plus rapide dans la modalité F2 expliquant le gain de temps observé par rapport à la modalité témoin. Même en l'absence de carence en azote assimilable, l'ajout d'extrait de levure, pendant la réhydratation, permet d'améliorer la fin de la fermentation alcoolique en influant sur la cinétique fermentaire (42° de pente de la courbe de fin de fermentation à comparer à 26° dans la fermentation témoin). Le résultat de l'exemple 1 observé sur moût synthétique est ainsi confirmé sur moût réel, à savoir que l'ajout d'extrait de levure dans le milieu de réhydratation permet une fermentation accélérée par rapport au même ajout dans le moût en début de fermentation alcoolique.

### Exemple 3 : Influence de l'ajout de différents nutriments sur la durée de fermentation d'un moût synthétique carencé

Douze fermentations ont été conduites en parallèle et en duplicata sur un moût synthétique MS 70-fa tel que décrit dans l'exemple 1, correspondant à un milieu carencé en azote assimilable (100 mg/l) et contenant 200 g/l de sucres fermentescibles. La levure sèche active utilisée est la levure commerciale Uvaferm CEG™, inoculée à la dose de 20 g/hl. L'extrait de levure est l'extrait commercial Bacto-yeast extract ref. 0127-01-7 (DIFCO, USA). Les vitamines et sels minéraux sont les produits standards disponibles auprès des fournisseurs.

Les douze fermentations correspondent aux modalités suivantes :
F1 et F2 : Témoins dans lesquels aucun extrait de levure n'est ajouté, ni dans le milieu de réhydratation de la levure ni dans le milieu de fermentation. Le milieu de réhydratation témoin est le milieu de base : eau glucosée à 50 g/l.
F3 : Fermentation conduite avec la levure CEG™ réhydratée en présence d'extrait de levure (utilisé à la dose permettant une concentration de 30g/hl dans le moût) dans le milieu de réhydratation.
F4 : Fermentation conduite avec la levure CEG™ réhydratée dans un milieu de réhydratation témoin. Le même extrait de levure que pour F3 est ajouté au début de la fermentation alcoolique directement dans le moût synthétique MS70-fa à la dose de 30 g/hl.
F5 : Fermentation conduite avec la levure CEG™ réhydratée en présence de levure inactive (LBI 2130™, Lallemand, Canada, utilisée à la dose permettant une concentration de 30g/hl dans le moût) dans le milieu de réhydratation
F6 : Fermentation conduite avec la levure CEG™ réhydratée dans un milieu de réhydratation témoin. La même levure inactive que pour F5 est ajoutée au début de la fermentation alcoolique directement dans le moût synthétique MS70-fa à la dose de 30 g/hl.
F7 : Fermentation conduite avec la levure CEG™ réhydratée en présence de phosphate diammonique (utilisé à la dose permettant une concentration de 10g/hl dans le moût) dans le milieu de réhydratation
F8 : Fermentation conduite avec la levure CEG™ réhydratée dans le milieu de réhydratation témoin. Le phosphate di-ammonique est ajouté en début de fermentation alcoolique à la dose de 10 g/hl.
F9 : Fermentation conduite avec la levure CEG™ réhydratée en présence d'un cocktail de vitamines (pantothénate, thiamine et biotine) dans le milieu de réhydratation de façon à obtenir des concentrations dans le moût de 60 µg/l, 34 µg/l, et 0,5 µg/l, respectivement.
F 10 : Fermentation conduite avec la levure CEG™ réhydratée dans le milieu de réhydratation témoin. Le cocktail de vitamines utilisé pour F9 est ajouté en début de fermentation alcoolique aux concentrations indiquées ci-dessus.
F11 : Fermentation conduite avec la levure CEG™ réhydratée en présence d'un cocktail de sels minéraux (Mg²⁺. Zn²⁺, Mn²⁺, sous forme de sulfates) dans le milieu de réhydratation de façon à obtenir des concentrations respectives des sels dans le moût de 460 µg/l, 58 µg/l et 4,5 µg/l.
F12 : Fermentation conduite avec la levure CEG™ réhydratée dans le milieu de réhydratation témoin. Le cocktail de sels minéraux utilisé pour F11 est ajouté en début de fermentation alcoolique, aux concentrations indiquées ci-dessus.

Les conditions de réhydratation sont identiques quel que soit le type de produit ajouté (extrait de levure, levure inactive, phosphate di-ammonique, cocktail de vitamines ou de sels minéraux). Elles correspondent à celles appliquées dans les exemples 1 et 2. Les volumes et conditions de fermentation sont identiques à ceux décrits dans les exemples 1 et 2. Les résultats obtenus sont consignés dans le tableau 1 ci-dessous. Les valeurs présentées correspondent à la moyenne des durées de fermentation en heures, des duplicata de chaque modalité.

**Tableau 1**

| | durée de fermentation , en heures | |
|---|---|---|
| Produit ajouté: | dans milieu réhydratation | en début de fermentation |
| 0 | 290 | 290 |
| extrait de levure | 250 | 280 |
| Levure inactive | 275 | 282 |
| Phosphate di-ammonique | 280 | 285 |
| Cocktail de vitamines | 283 | 288 |
| Cocktail de sels | 265 | 270 |

Les résultats montrent que quel que soit le type d'élément nutritif ajouté, les additions durant la phase de réhydratation permettent une meilleure réduction des durées de fermentation que les mêmes additions réalisées en début de fermentation alcoolique.

### Exemple 4 : Composition sèche et milieu de réhydratation M1 correspondant

Composition sèche en poudre prête à l'emploi :

| | |
|---|---|
| levure inactive | 998,10 g |
| phosphate di-ammonique | 333,33 g |
| acide pantothénique | 200,00 mg |
| thiamine | 113,33 mg |
| biotine | 1,65 mg |
| sulfate de zinc | 195,00 mg |
| sulfate de magnésium | 1 500,00 mg |
| sulfate de manganèse | 15,00 mg |

Une quantité de 150 g de cette composition sèche est pesée et est introduite dans 1 litre d'eau glucosée à 50 g/l. On obtient le milieu de réhydratation M1 suivant:

| | |
|---|---|
| levure inactive | 149,71 g/l |
| phosphate di-ammonique | 50,00 g/l |
| acide pantothénique | 30,00 mg/l |
| thiamine | 17,00 mg/l |
| biotine | 0,25 mg/l |
| sulfate de zinc | 29,25 mg/l |
| sulfate de magnésium | 225,00 mg/l |
| sulfate de manganèse | 2,25 mg/l |

### Exemple 5 : Procédé de réhydratation d'une levure de vinification commerciale

On introduit 15 g de levure sèche active Lalvin EC1118^{™} dans 100 ml de milieu de réhydratation M1 préparé suivant l'exemple 4. Après incubation 30 minutes à 37°C, on obtient un inoculum prêt à ensemencer un moût à raison de 20 ml d'inoculum par litre. Les concentrations en nutriments apportées dans le moût sont ainsi les suivantes (on considère que le volume inoculé contient lesdits éléments soit sous forme libre dans la phase aqueuse du milieu de réhydratation, soit sous forme assimilée par les levures réhydratées):

| | |
|---|---|
| phosphate di-ammonique | 0,10 g/l |
| acide pantothénique | 60,00 µg/l |
| thiamine | 34,00 µg/l |
| biotine | 0,50 µg/l |
| sel de zinc | 0,058 µg/l |
| sel de magnésium | 0,45 µg/l |
| sel de manganèse | 4,50 µg/l |

### Exemple 6 : Milieu de réhydratation M2 et composition C2.

Dans 1 litre d'eau purifiée, on mélange 50 g de glucose et 150 g de la composition sèche C2, comprenant:

| | |
|---|---|
| levures inactives | 149,49 g |
| pantothénate de calcium | 27,0 mg |
| biotine | 0,2 mg |
| sel de zinc | 50,0 mg |
| sel de magnésium | 433,0 mg |
| sel de manganèse | 4,0 mg |

Dans ce milieu M2, seront réhydratés 100 g de levure sèche active Uvaferm CEG_{™}, commercialisée par Lallemand. Le milieu M2 contenant la levure réhydratée sera inoculé dans un moût à fermenter à raison de 0,08 à 0,41/hl, de préférence à 0,21/hl.

### Exemple 7 : Application à la préparation d'une boisson fermentée

Le milieu M2 contenant la composition C2 a été utilisé pour réhydrater des LSA et ensemencer un moût de fermentation d'un vin blanc en conditions industrielles dans une cave de la région de la Mancha en Espagne.
Cépage : Airen
Azote assimilable dans le moût : 225 mg/l (moût non carencé)

Des fermentations ont été réalisées dans des cuves de 100 hectolitres selon différentes modalités, afin de déterminer les conditions le plus favorables. Deux types de LSA ont été employées, seules ou en présence d'autres substances communément ajoutées aux moûts pour favoriser la fermentation. En particulier a été étudiée l'influence du phosphate de diammonium et d'un activateur de fermentation, apportés dans le milieu de fermentation.

Les levures sèches actives Uvaferm™ CEG (Lallemand, Canada) et Uvaferm™ PM (Lallemand, Canada), ont été réhydratées à l'aide du milieu M2 décrit à l'exemple 6. Pour chacune des deux levures, 4 essais ont été conduits, en ajoutant :

| | | | |
|---|---|---|---|
| Essai N°1 | Phosphate diammonique | 0,2 g/l | début de fermentation |
| | | | |
| Essai N°2 | Phosphate diammonique | 0,2 g/l | début de fermentation |
| | Activateur de fermentation | 0,3 g/l | premier tiers de la fermentation |
| | | | |
| Essai N° 3 | Composition C2 | 0,3 g/l (équiv) | milieu de réhydratation |
| | Phosphate diammonique | 0,2 g/l | début de fermentation |
| | | | |
| Essai N°4 | Composition C2 | 0,3 g/l (équiv) | milieu de réhydratation |
| | Activateur de fermentation | 0,3 g/l | premier tiers de la fermentation |

L'activateur de fermentation utilisé est l'activateur Fermaid™ (Lallemand, Canada).

Le profil cinétique de ces huit fermentations a été suivi par mesure de la densité du moût et au niveau de la production d'un composé secondaire de la fermentation, à savoir l'acétate. En effet, l'acétate est responsable de l'acidité volatile des vins, qui doit être minimisée pour obtenir de bonnes qualités gustatives. Les résultats obtenus sont consignés dans les tableaux 2 et 3 ci-dessous.

Les valeurs présentées dans le tableau 2 correspondent aux durées de fermentation en jours de chaque modalité.

**Tableau 2**

| | durée de fermentation, en jours | |
|---|---|---|
| N° Essai | Uvaferm^{™} CEG | Uvaferm^{™} PM |
| 1 | 10 | 8 |
| 2 | 9 | 7 |
| 3 | 9 | 7 |
| 4 | 9 | 6 |

Il apparaît que pour les deux levures sèches actives testées, l'addition de la composition C2 dans le milieu de réhydratation permet d'améliorer sensiblement le profil de la cinétique de fermentation (Essai N°3 comparé à Essai N°1). De plus, comme on le voit avec l'Essai N°4 dans lequel on obtient les cinétiques les plus rapides, l'effet obtenu par l'ajout de la composition C2 dans le milieu de réhydratation des LSA agit de manière cumulative avec l'effet produit par l'addition d'un activateur de fermentation.

Les valeurs présentées dans le tableau 3 correspondent à l'acidité volatile mesurée en fin de fermentation par la méthode de Duclaux -Gayon, connue de l'homme du l'art.

**Tableau 3**

| | Acidité volatile, en g/l | |
|---|---|---|
| N° Essai | Uvaferm^{™} CEG | Uvaferm^{™} PM |
| 1 | 0,48 | 0,33 |
| 2 | 0,51 | 0,33 |
| 3 | 0,44 | 0,18 |
| 4 | 0,46 | 0,22 |

Il apparaît au vu de ces résultats que l'addition de la composition C2 durant la réhydratation permet de diminuer la production d'acidité volatile. Ceci pourrait être du au fait que les levures sont dans de meilleures conditions physiologiques et ainsi aptes à mieux supporter les conditions physico-chimiques rencontrées dans un moût en fermentation. Cet état physiologique se traduirait alors par une meilleure résistance aux stress, induisant une production moindre d'acidité volatile.

Cet effet positif supplémentaire renforce l'intérêt du procédé selon la présente invention pour la réhydratation de LSA destinées à la fermentation alcoolique. De manière générale, les résultats obtenus à l'issue de ces essais confirment cet intérêt à l'échelle industrielle.

## Revendications

1. Procédé de réhydratation de levures sèches actives comprenant essentiellement les étapes consistant à :
a) préparer un milieu de réhydratation aqueux, sucré ou non sucré, comprenant
i) des levures inactives ou un dérivé de levure à raison de 100 à 200 g l, de préférence 150 g l de milieu, et éventuellement
ii) un ou plusieurs nutriments choisis parmi des sources d'azote organique comprenant des sels d'ammonium, des nitrates, l'urée, des acides aminés, des peptides, des protéines ou une source biologique riche en azote : des acides gras, des stérols, une combinaison de ceux-ci ou une source naturelle riche en ces éléments telle que notamment les bourbes : des vitamines comprenant la thiamine, la biotine, l'acide pantothénique, la niacine, la riboflavine, la pyridoxine, ou une source naturelle riche en vitamines : des sels minéraux comprenant des phosphates, des sels de zinc. de magnésium, de calcium, de potassium, de sodium, de fer, de cuivre, de manganèse ou une combinaison de ces sels minéraux, chaque nutriment choisi étant apporté à une concentration 200 à 1 000 fois supérieure, de préférence 500 fois supérieure. à celle qu'il aura dans le moût à fermenter, dans un milieu aqueux.
b) introduire les levures sèches actives dans le milieu de réhydratation ainsi préparé à raison de 50 à 150 g l, de préférence 100 g l de milieu,
c) incuber le milieu de réhydratation contenant les levures sèches actives à une température comprise entre 30°C et 45°C, de préférence 37°C, durant 20 à 40 minutes, de préférence 30 minutes.

2. Procédé de réhydratation de levures sèches actives selon la revendication 1, comprenant essentiellement les étapes consistant à :
a) Préparer un milieu de réhydratation comprenant.
i) des levures inactives ou un dérivé de levure à raison de 100 à 200 g l, de préférence 150 gl de milieu, et
ii) un ou plusieurs des composants suivants : 20 à 50 mg l de pantothénate de calcium, 0.15 à 0.30 mg l de biotine.
20 à 60 mg l de sel de zinc, 200 à 500 mg l de sel de magnésium, 2 à 5 mg l de sel de manganèse, dans un milieu aqueux.
b) introduire les levures sèches actives dans le milieu de réhydratation ainsi préparé à raison de 50 à 150 g l, de préférence 100 g l de milieu.
c) incuber le milieu de réhydratation contenant les levures sèches actives à une température comprise entre 30°C et 45°C, de préférence 37°C, durant 20 à 40 minutes, de préférence 30 minutes.

3. Procédé selon l'une quelconque des revendication 1 à 2, **caractérisé en ce que** les levures sèches actives sont des levures de vinification.

4. Procédé selon l'une quelconque des revendication 1 à 3, **caractérisé en ce que** les levures sèches actives sont des *Saccharomyces.*

5. Procédé selon la revendication 4. **caractérisé en ce que** les *Saccharomyces* sont du genre *cerivisiae.*

6. Procédé selon l'une quelconque des revendications 1 à 5, **caractérisé en ce que** les levures inactives sont des levures enrichies en nutriments.

7. Procédé selon l'une quelconque des revendications 1 à 5, **caractérisé en ce que** les levures inactives sont enrichies en sels minéraux.

8. Procédé selon l'une quelconque des revendications 1 à 7, **caractérisé en ce que** les dérivés de levure sont des produits susceptibles d'être obtenus à partir de cellules de levures enrichies ou fractionnées, par action physique ou chimique.

9. Procédé selon la revendication 8, **caractérisé en ce que** lesdits produits sont des extraits de levures obtenus par autolyse et des écorces de levure.

10. Milieu de réhydratation de levures sèches actives comprenant au moins, dans un milieu aqueux, sucré ou non, des levures inactives ou un dérivé de levure à raison de 100 à 200 g l, de préférence 150 g l de milieu.

11. Milieu de réhydratation selon la revendication 10 comprenant au moins, dans un milieu aqueux, sucré ou non.
i) des levures inactives ou un dérivé de levure, à raison de 100 à 200 g l, de préférence 150 g l de milieu.
ii) un ou plusieurs nutriments choisis parmi des sources d'azote organique comprenant des sels d'ammonium, l'urée, des acides aminés, des peptides, des protéines ou une source biologique riche en azote : des acides gras, des stérols, une combinaison de ceux-ci ou une source naturelle riche en ces éléments telle que notamment les bourbes : des vitamines comprenant la thiamine, la biotine, l'acide pantothénique, la niacine, la riboflavine, la pyridoxine, ou une source naturelle riche en vitamines : des sels minéraux comprenant des phosphates, nitrates, des sels de zinc, de magnésium, de calcium, de potassium, de sodium, de fer, de cuivre, de manganèse, ou une combinaison de ces sels minéraux, chaque nutriment choisi étant apporté à une concentration 200 à 1 000 fois supérieure, de préférence 500 fois supérieure, à celle qu'il aura dans le moût à fermenter.

12. Milieu de réhydratation de levures sèches actives selon la revendication 11, comprenant au moins, dans un milieu aqueux, de préférence de l'eau glucosée à raison de 50 g l ou du jus de raisin.
i) des levures inactives ou des extraits de levure, à raison de 100 à 200 g l, de préférence 150 g l.
ii) un ou plusieurs des composants suivants : 27 mg l de pantothénate de calcium, 0.2 mg l de biotine, 50 mg l de sel de zinc, 433 mg l de sel de magnésium, 4 mg l de sel de manganèse.

13. Utilisation d'une composition sèche pour la préparation d'un milieu de réhydratation de levures sèches actives, **caractérisée en ce qu'**elle comprend au moins des levures inactives ou des dérivés de levure ajoutées dans le milieu de réhydratation à raison de 100 à 200 g l, de préférence 150 g l, sous forme déshydratée et un ou plusieurs nutriments choisis parmi des sources d'azote organique comprenant des sels d'ammonium, des acides aminés, des peptides, des protéines ou une source biologique riche en azote : des acides gras, des stérols, une combinaison de ceux-ci ou une source naturelle riche en ces éléments telle que notamment les bourbes : des vitamines comprenant la thiamine, la biotine, l'acide pantothénique, la niacine, la riboflavine, la pyridoxine, ou une source naturelle riche en vitamines : des sels minéraux comprenant des phosphates, des sels de zinc, de magnésium, de calcium, de potassium, de sodium, de fer, de cuivre, de manganèse ou une combinaison de ces sels minéraux.

14. Composition sèche destinée à la préparation d'un milieu de réhydratation de levures sèches actives selon l'une quelconque des revendications 10 à 12, **caractérisée en ce qu'**elle comprend au moins.
i) environ 99.3 % en poids de levures inactives ou de dérivé de levure, sous forme déshydratée.
ii) un ou plusieurs nutriments choisis parmi l'acide pantothénique, la biotine, un sel de zinc, un sel de magnésium, un sel de manganèse.

15. Procédé de production d'une boisson fermentée alcoolisée, **caractérisé en ce qu'**il comprend:
- la réhydratation de levures sèches actives selon le procédé de l'une quelconque des revendications 1-9,
- l'ensemencement de moût par les levures réhydratées ainsi obtenues.

16. Procédé de production d'une boisson fermentée alcoolisée, **caractérisé en ce qu'**il comprend:
- la réhydratation de levures sèches actives dans un milieu de réhydratation selon l'une quelconque des revendications 10-12.
- l'ensemencement de moût par les levures réhydratées ainsi obtenues.

17. Procédé de production d'une boisson fermentée alcoolisée selon l'une quelconque des revendications 15 ou 16, **caractérisé en ce que** le moût est un jus de raisin.

18. Inoculum de levures, **caractérisé en ce qu'**il contient les levures sèches actives revivifiées selon le procédé de l'une quelconque des revendications 1 à 9 et du milieu de réhydratation selon l'une quelconque des revendications 10 à 12.

## Patentansprüche

1. Verfahren zur Rehydratation von aktiven Trockenhefen, das im Wesentlichen aus folgenden Schritten besteht:
**a)** Herstellen eines wässrigen, zuckerhaltigen oder nichtzuckerhaltigen Rehydratationsmedium, umfassend:
i) inaktive Hefen oder ein Hefederivat in einer Menge von 100 bis 200 g/l Medium, vorzugsweise 150 g/l Medium und gegebenenfalls:
ii) einen oder mehrere Nährstoffe, die ausgewählt sind aus organischen Stickstoffquellen mit Ammoniumsalzen, Nitraten, Harnstoff, Aminosäuren, Peptiden, Proteinen oder einer biologischen Quelle mit hohem Stickstoffanteil; Fettsäuren, Sterole, einer Kombination von beidem oder einer natürlichen Quelle mit einem hohen Anteil dieser Elemente wie insbesondere Trub; Vitamine mit Thiamin, Biotin, Panthotensäure, Niacin, Riboflavin, Pyridoxin oder einer natürlichen Quelle mit hohem Vitamingehalt; Mineralsalzen mit Phosphaten, Zinksalzen, Magnesiumsalzen, Calciumsalzen, Kaliumsalzen, Natriumsalzen, Eisensalzen, Kupfersalzen, Mangansalzen oder einer Kombination dieser Mineralsalze, wobei jeder ausgewählte Nährstoff in einer 200 bis 1000 mal höheren, vorzugsweise 500 mal höheren Konzentration als der im zu gärenden Most vorhandenen Konzentration hinzugefügt wird, in einem wässrigen Medium,
**b)** Einführen der aktiven Trockenhefen in dem so hergestellten Rehydratationsmedium in einer Menge von 50 bis 150 g/l Medium, vorzugsweise 100 g/l Medium,
**c)** Inkubieren des Rehydratationsmedium, das die aktiven Trockenhefen enthält, bei einer Temperatur zwischen 30°C und 45°C, vorzugsweise bei 37°C, während von 20 bis 40 Minuten, vorzugsweise 30 Minuten.

2. Verfahren zur Rehydratation aktiven Trockenhefen gemäß Anspruch 1, das im Wesentlichen aus folgenden Schritten besteht:
a) Herstellen eines Rehydratationsmedium, umfassend:
i) inaktive Hefen oder ein Hefederivat in einer Menge von 100 bis 200 g/l Medium, vorzugsweise 150 g/l Medium,
und
ii) einen oder mehrere der folgenden Bestandteile: 20 bis 50 mg/l Calciumpantothenat, 0,15 bis 0,30 mg/l Biotin, 20 bis 60 mg/l Zinksalz, 200 bis 500 mg/l Magnesiumsalz, 2 bis 5 mg/l Mangansalz, in einem wässrigen Medium,
b) Einführen der aktiven Trockenhefen in dem so hergestellten Rehydratationsmedium in einer Menge von 50 bis 150 g/l Medium, vorzugsweise 100 g/l Medium,
c) Inkubieren des Rehydratationsmediums, das die aktiven Trockenhefen enthält, bei einer Temperatur zwischen 30°C und 45°C, vorzugsweise bei 37°C, während von 20 bis 40 Minuten, vorzugsweise 30 Minuten.

3. Verfahren nach einem der Ansprüche 1 bis 2, das **dadurch gekennzeichnet ist, dass** es sich bei den aktiven Trockenhefen um Weinhefen handelt.

4. Verfahren nach einem der Ansprüche 1 bis 3, das **dadurch gekennzeichnet ist, dass** es sich bei den aktiven Trockenhefen um *Saccharomyces* handelt.

5. Verfahren nach Anspruch 4, das **dadurch gekennzeichnet ist, dass** es sich bei den *Saccharomyces* um die Spezies *cerivisiae* handelt.

6. Verfahren nach einem der Ansprüche 1 bis 5, das **dadurch gekennzeichnet ist, dass** die inaktiven Hefen mit Nährstoffen angereichert sind.

7. Verfahren nach einem der Ansprüche 1 bis 5, das **dadurch gekennzeichnet ist, dass** die inaktiven Hefen mit Mineralsalzen angereichert sind.

8. Verfahren nach einem der Ansprüche 1 bis 7, das **dadurch gekennzeichnet ist, dass** die Hefederivate Produkte sind, die durch physikalische oder chemische Einwirkung aus angereicherten oder fraktionierten Hefezellen erhalten werden können.

9. Verfahren nach Anspruch 8, das **dadurch gekennzeichnet ist, dass** es sich bei den besagten Produkte um Hefeextrakte , die durch Autolyse erhalten werden oder um Hefezellwände handelt.

10. Medium zur Rehydratation aktiver Trockenhefe, das in einem wässrigen, zuckerhaltigen oder nichtzuckerhaltigen Medium wenigstens inaktive Hefen oder ein Hefederivat in einer Menge von 100 bis 200 g/l Medium, vorzugsweise 150 g/l Medium umfasst.

11. Rehydratationsmedium nach Anspruch 10, das in einem wässrigen, zuckerhaltigen oder nichtzuckerhaltigen Medium wenigstens Folgendes umfasst:
**i)** inaktive Hefen oder ein Hefederivat in einer Menge von 100 bis 200 g/l Medium, vorzugsweise 150 g/l Medium,
**ii)** einen oder mehrere Nährstoffen, die ausgewählt sind aus organischen Stickstoffquellen mit Ammoniumsalzen, Harnstoff, Aminosäuren, Peptiden, Proteinen oder einer biologischen Quelle mit hohem Stickstoffanteil; Fettsäure, Sterole, einer Kombination von beidem oder einer natürlichen Quelle mit einem hohen Anteil dieser Elemente wie insbesondere Trub; Vitamine mit Thiamin, Biotin, Panthotensäure, Niacin, Riboflavin, Pyfridoxin oder eine natürliche Quelle mit hohem Vitamingehalt; Mineralsalze mit Phosphaten, Nitraten, Zinksalze, Magnesimnsalze, Calciumsalze, Kaliumsalze, Natriumsalze, Eisensalze, Kupfersalze, Mangansalze oder eine Kombination dieser Mineralsalze, wobei jeder ausgewählte Nährstoff in einer 200 bis 1000 mal höheren, vorzugsweise 500 mal höheren Konzentration als der im zu gärenden Most vorhandenen Konzentration hinzugefügt wird.

12. Rehydratationsmedium für aktive Trockenhefen nach Anspruch 11, das in einem wässrigen Medium, vorzugsweise 50 g/l Glucose in Wasser oder Traubensaft, wenigstens Folgendes umfasst:
**i)** inaktive Hefen oder ein Hefeextrakte in einer Menge von 100 bis 200 g/l Medium, vorzugsweise 150 g/l Medium,
**ii)** einen oder mehrere der folgenden Bestandteile: 27 mg/l Calciumpantothenat, 0,2 mg/l Biotin, 50 mg/l Zinksalz, 433 mg/l Magnesiumsalz, 4 mg/l Mangansalz.

13. Verwendung einer trockenen Zusammensetzung für die Zubereitung eines Rehydratationsmedium für aktive Trockenhefen, die **dadurch gekennzeichnet ist, dass** sie wenigstens Folgendes umfasst: inaktive Hefen oder Hefederivate, die in einer Menge von 100 bis 200 g/l, vorzugsweise 150 g/l in dehydrierter Form in das Rehydratationsmedium gegeben werden, und einen oder mehrere Nährstoffe, die ausgewählt sind aus organischen Stickstoffquellen mit Ammoniumsalzen, Aminosäuren, Peptiden, Proteinen oder einer biologischen Quelle mit hohem Stickstoffanteil; Fettsäure, Sterole, einer Kombination von beidem oder einer natürlichen Quelle mit einem hohen Anteil dieser Elemente wie insbesondere Trub; Vitamine mit Thiamin, Biotin, Panthotensäure, Niacin, Riboflavin, Pyridoxin oder einer natürlichen Quelle mit hohem Vitamingehalt; Mineralsalze mit Phosphaten, Nitraten, Zinksalze, Magnesiumsalze, Calciumsalze, Kaliumsalze, Natriumsalze, Eisensalze, Kupfersalze, Mangansalze oder eine Kombination dieser Mineralsalze.

14. Trockene Zusammensetzung für die Zubereitung eines Rehydratationsmedium für aktive Trockenhefe nach einem der Ansprüche 10 bis 12, die **dadurch gekennzeichnet ist, dass** sie wenigstens Folgendes umfasst:
i) ca. 99,3 % ihres Gewichts an inaktiven Hefen oder Hefederivaten in dehydrierter Form,
ii) einen oder mehrere Nährstoffe, gewählt aus Pantothensäure, Biotin, ein Zinksalz, ein Magnesiumsalz, ein Mangansalz.

15. Verfahren zur Produktion eines fermentierten alkoholischen Getränks, das **dadurch gekennzeichnet ist, dass** es Folgendes umfasst:
- die Rehydratation von aktiven Trockenhefen nach dem Verfahren eines der Ansprüche 1-9,
- die Aussaat des Mostes mit den derart erhaltenen rehydrierten Hefen

16. Verfahren zur Produktion eines fermentierten alkoholischen Getränks, das **dadurch gekennzeichnet ist, dass** es Folgendes umfasst:
- die Rehydratation von aktiven Trockenhefen in einem Rehydratationsmedium nach einem der **Ansprüche 10-12,**
- die Aussaat des Mostes mit den derart erhaltenen rehydrierten Hefen

17. Verfahren zur Produktion eines fermentierten alkoholischen Getränks nach einem der Ansprüche 15 oder 16, das **dadurch gekennzeichnet ist, dass** der Most ein Traubensaft ist.

18. Inokulum von Hefen, das **dadurch gekennzeichnet ist, dass** es aktive Trockenhefen, die nach dem Verfahren nach einem der Ansprüche 1 bis 9 wiederbelebten wurden, und Rehydratationsmedium nach einem der Ansprüche 10 bis 12 enthält.

## Claims

1. A method of rehydrating active dry yeasts, essentially comprising the steps consisting in :
a. Preparing a rehydratation aqueous medium, which is or is not sugary, comprising
i. inactive yeasts or a yeast derivative, at a rate of 100 to 200g/l, preferably 150g/l of medium, and optionally
ii. one or more nutrients chosen from organic nitrogen sources comprising ammonium salts, nitrates, urea, amino acids, peptides, proteins or a biological source rich in nitrogen ; fatty acids, sterols, a combination thereof or a natural source rich in these elements such as especially mud ; vitamins comprising thiamine, biotin, pantothenic acid, niacin, riboflavin, pyridoxine, or a natural source rich in vitamins ; mineral salts comprising phosphates, salts of zinc, of magnesium, of calcium, of potassium, of sodium, of iron, of copper, of manganese or a combination of these mineral salts, each selected nutrient being added at a concentration which is 200 to 1000 time greater, preferably 500 time greater than, that which it will have in the must to be fermented, in an aqueous medium,
b. Introducing the active dry yeasts into the rehydration medium thus prepared at a rate of 50 to 150g/l, preferably 100g/l of medium,
c. Incubating the rehydration medium containing the active dry yeasts at a temperature comprised between 30°C and 45°C, preferably 37°C, for 20 to 40 minutes, preferably 30 minutes.

2. The method of rehydrating active dry yeasts according to claim 1, essentially comprising the steps consisting in :
a. Preparing a rehydration medium comprising,
i. inactive yeasts or a yeast derivative, at a rate of 100 to 200g/l, preferably 150g/l of medium, and
ii. one or more of the following components : 20 to 50mg/l of calcium pantothenate, 0,15 to 0,30mg/l of biotin, 20 to 60 mg/l of zinc salt, 200 to 500mg/l of magnesium salt, 2 to 5 mg/l of manganese salt, in an aqueous medium,
b. Introducing the active dry yeasts into the rehydration medium thus prepared at a rate of 50 to 150g/l, preferably 100g/l of medium,
c. Incubating the rehydration medium containing the active dry yeasts at a temperature comprised between 30°C and 45°C, preferably 37°C, for 20 to 40 minutes, preferably 30 minutes.

3. The method according to anyone of claims 1 to 2, wherein the active dry yeasts are vinification yeasts.

4. The method according to anyone of claims 1 to 3, wherein the active dry yeasts are *Saccharomyces.*

5. The method according to claim 4, wherein the *Saccharomyces* are of *cerevisiae* genus.

6. The method according to anyone of claims 1 to 5, wherein the inactive yeasts are nutrient-enriched yeasts.

7. The method according to anyone of claims 1 to 5, wherein the inactive yeasts are enriched in mineral salts.

8. The method according to anyone of claims 1 to 7, wherein yeasts derivatives are products susceptible to be obtained from enriched or fractionated yeasts cells, by physical or chemical action.

9. The method according to claim 8, wherein said products are yeasts extracts obtained by autolysis and yeast cell walls.

10. A rehydration medium for active dry yeasts comprising at least, in an aqueous medium, which is or not sugary, inactive yeasts or yeast derivatives at the rate of 100 to 200g/l, preferably 150g/l of medium.

11. The rehydration medium according to claim 10 comprising at least, in an aqueous medium, which is or not sugary,
i. inactive yeasts or a yeast derivative, at the rate of 100 to 200g/l, preferably 150g/l of medium,
ii. one or more nutrients chosen from organic nitrogen sources comprising ammonium salts, urea, amino acids, peptides, proteins or a biological source rich in nitrogen ; fatty acids, sterols, a combination thereof or a natural source rich in these elements such as especially mud ; vitamins comprising thiamine, biotin, pantothenic acid, niacin, riboflavin, pyridoxine, or a natural source rich in vitamins ; mineral salts comprising phosphates, nitrates, salts of zinc, of magnesium, of calcium, of potassium, of sodium, of iron, of copper, of manganese or a combination of these mineral salts, each selected nutrient being added at a concentration which is 200 to 1000 time greater, preferably 500 time greater than, that which it will have in the must to be fermented.

12. The rehydration medium for active dry yeasts according to claim 11, comprising at least, in an aqueous medium, preferably water with glucose, at the rate of 50g/l or grape juice,
i. inactive yeasts or a yeast extracts, at the rate of 100 to 200g/l, preferably 150g/l,
ii. one or more of the following components : 27 mg/l of calcium pantothenate, 0,2 mg/l of biotin, 50 mg/l of zinc salt, 43 3mg/l of magnesium salt, 4 mg/l of manganese salt.

13. Use of a dry composition for the preparation of a rehydration medium for active dry yeasts, wherein the dry composition comprises at least inactive yeasts or yeasts derivatives added to the rehydration medium at the rate of 100 to 200 g/l, preferably 150g/l, in dehydrated form et one or more nutrients chosen from organic nitrogen sources comprising ammonium salts, amino acids, peptides, proteins or a biological source rich in nitrogen ; fatty acids, sterols, a combination thereof or a natural source rich in these elements such as especially mud ; vitamins comprising thiamine, biotin, pantothenic acid, niacin, riboflavin, pyridoxine, or a natural source rich in vitamins ; mineral salts comprising phosphates, salts of zinc, of magnesium, of calcium, of potassium, of sodium, of iron, of copper, of manganese or a combination of these mineral salts.

14. Dry composition intented to the preparation of a rehydration medium for active dry yeasts according to anyone of claims 10 to 12, wherein the dry composition comprises at least,
i. about 99,3% by weight of inactive yeasts or yeast derivative, in dehydrated form,
ii. one or more nutrients chosen among pantothenic acid, biotin, zinc of salt, magnesium salt, manganese salt.

15. A method for producing a fermented alcoholic drink, comprising:
- the rehydration of active dry yeasts according to the method of anyone of claims 1 to 9,
- the seeding of the must with the thus obtained rehydrated yeasts.

16. A method for producing a fermented alcoholic drink, comprising:
- the rehydration of active dry yeasts in a rehydration medium according to anyone of claims 10 to 12,
- the seeding of the must with the thus obtained rehydrated yeasts.

17. A method for producing a fermented alcoholic drink according to anyone of claims 15 or 16, wherein the must is a grape juice.

18. Yeast inoculum **characterized in that** it contains revivified active dry yeasts according to the method of any one of claims 1 to 9 and the rehydration medium according to any one of claims 10 to 12.
